# EUROPÄISCHE PATENTSCHRIFT

(11) **EP 1 480 646 B1**
(45) Veröffentlichungstag und Bekanntmachung des Hinweises auf die Patenterteilung: **30.07.2008**
(21) Anmeldenummer: 03714776.6
(22) Anmeldetag: 05.03.2003
(51) Int. Cl.: A61K 31/4709, A61K 31/43, A61K 31/403, A61K 31/198, A61K 31/138, A61K 38/21, A61P 9/10, A61P 31/00

(54) **ANTIINFEKTIVA UND/ODER IMMUNMODULATOREN ZUR PRÄVENTIVEN THERAPIE NACH AKUTEM SCHLAGANFALL**
ANTI-INFECTIVE AGENTS AND/OR IMMUNOMODULATORS USED FOR PREVENTIVE THERAPY FOLLOWING AN ACUTE CEREBROVASCULAR ACCIDENT
AGENTS ANTI-INFECTIEUX ET/OU IMMUNOMODULATOIRES DESTINES A LA THERAPIE PREVENTIVE A LA SUITE D'UN ACCIDENT CEREBROVASCULAIRE GRAVE

(30) Priorität: 05.03.2002 DE 10210536; 24.04.2002 DE 10218328
(43) Veröffentlichungstag der Anmeldung: 01.12.2004
(73) Patentinhaber: Charité - Universitätsmedizin Berlin, 10117 Berlin (DE)
(72) Erfinder: MEISEL, Andreas, 13127 Berlin (DE); PRASS, Konstantin, 16259 Zächericker Loose (DE); MEISEL, Christian, Oxfor d OX2 7SW (GB); HALLE, Elke, 10785 Berlin (DE); DIRNAGL, Ulrich, 10719 Berlin (DE); VOLK, Hans, Dieter, 10178 Berlin (DE)
(74) Vertreter: Krauss, Jan
(86) Internationale Anmeldenummer: PCT/EP2003/002246
(87) Internationale Veröffentlichungsnummer: WO 2003/074057

(56) Entgegenhaltungen:
- WO-A-01/10465
- WO-A-96/12500
- GIROIR B P ET AL: "Preliminary evaluation of recombinant amino-terminal fragment of human bactericidal/permeability-increasing protein in children with severe meningococcal sepsis" LANCET, XX, XX, Bd. 350, Nr. 9089, 15. November 1997 (1997-11-15), Seiten 1439-1443, XP004264991 ISSN: 0140-6736
- "Application of a new fluoroquinolone, moxifloxacin (Avalox(R)) for the treatment of chronic bronchitis" NOTFALL MEDIZIN 1999 GERMANY, Bd. 25, Nr. 11, 1999, Seite 478 XP009013170 ISSN: 0341-2903
- YRJANHEIKKI JUHA ET AL: "A tetracycline derivative, minocycline, reduces inflammation and protects against focal cerebral ischemia with a wide therapeutic window." PROCEEDINGS OF THE NATIONAL ACADEMY OF SCIENCES OF THE UNITED STATES, Bd. 96, Nr. 23, 9. November 1999 (1999-11-09), Seiten 13496-13500, XP002246594 Nov. 9, 1999 ISSN: 0027-8424
- ALLEWELT M ET AL: "[Diagnosis and therapy of abscess forming pneumonia]" THERAPEUTISCHE UMSCHAU. REVUE THERAPEUTIQUE. SWITZERLAND OCT 2001, Bd. 58, Nr. 10, Oktober 2001 (2001-10), Seiten 599-603, XP009013149 ISSN: 0040-5930
- DATABASE WPI Section Ch, Week 199908 Derwent Publications Ltd., London, GB; Class B04, AN 1999-081970 XP002246596 & CN 1 194 828 A (WANG W), 7. Oktober 1998 (1998-10-07)
- FONG I W: "Antibiotics effects in a rabbit model of Chlamydia pneumoniae-induced atherosclerosis." THE JOURNAL OF INFECTIOUS DISEASES. UNITED STATES JUN 2000, Bd. 181 Suppl 3, Juni 2000 (2000-06), Seiten S514-S518, XP009013134 ISSN: 0022-1899
- PANELLA P ET AL: "[Monitoring of low urinary tract infections in patients hospitalized with neurological diseases]" ARCHIVIO ITALIANO DI UROLOGIA, ANDROLOGIA: ORGANO UFFICIALE [DI] SOCIETA ITALIANA DI ECOGRAFIA UROLOGICA E NEFROLOGICA / ASSOCIAZIONE RICERCHE IN UROLOGIA. ITALY DEC 1994, Bd. 66, Nr. 5, Dezember 1994 (1994-12), Seiten 259-264, XP009013147 ISSN: 1124-3562
- DAVENPORT R J ET AL: "Complications after acute stroke." STROKE, [Online] Bd. 27, Nr. 3, 1996, Seiten 415-420, XP002246595 ISSN: 0039-2499 Gefunden im Internet: <URL:http://stroke.ahajournals.org/cgi/con tent/full/27/3/415> [gefunden am 2003-06-18]
- MARTYNOV YU S ET AL: "EARLY AND LATE POST STROKE PNEUMONIAS" ZHURNAL NEVROPATOLOGII I PSIKHIATRII IMENI S S KORSAKOVA, Bd. 80, Nr. 11, 1980, Seiten 1628-1634, XP009013167 ISSN: 0044-4588
- CHRYSOS G E ET AL: "Hospital infections in diabetic patients with cerebral stroke." INTERNATIONAL JOURNAL OF ANTIMICROBIAL AGENTS, Bd. 17, Nr. Supplement 1, Juni 2001 (2001-06), Seite S72 XP001152998 22nd International Congress of Chemotherapy;Amsterdam, Netherlands; June 30-July 03, 2001 ISSN: 0924-8579

## Beschreibung

Die Erfindung betrifft Mittel zur präventiven antiinfektiven Therapie nach akutem Schlaganfall, mit dem Ziel, die Letalität und Morbidität nach dem Schlaganfall durch die Verhinderung von Infektionen nach akutem Schlaganfall zu vermindern. Infektionen sind schwere Komplikationen, die regelhaft in der frühen Phase nach akutem Schlaganfall auftreten und einen prognostisch negativen Einfluss haben. Bei den erfindungsgemäß in pharmazeutischen Zubereitungen eingesetzten Mitteln handelt es sich um Antiinfektiva, vor allem Antibiotika, die zur Prävention und Behandlung von Pneumonien, Harnwegsinfektionen und Sepsis bei Säugetieren, besonders bei Haus- und Nutztieren sowie insbesondere auch beim Menschen eingesetzt werden. Die Erfindung betrifft weiterhin Mittel zur immunmodulatorischen Therapie nach akutem Schlaganfall, mit dem Ziel; Infektionen nach dem Schlaganfall zu verhindern. Bei den erfindungsgemäß in pharmazeutischen Zubereitungen eingesetzten Mitteln handelt es sich um Substanzen, die immunmodulatorische Potenzen besitzen.

Der akute Schlaganfallpatient ist neben den direkten Folgen des Schlaganfalls, die von transitorischen über permanente neurologische Ausfälle bis zum Tod infolge Hirndrucks reichen können, insbesondere in der Akut- und frühen Remissionsphase vor allem durch Infektionen gefährdet. Infektionen und insbesondere Pneumonien stellen die Hauptursache für die Letalität beim Schlaganfall dar (Henon et al. 1995, Katzan et al. 2003, Literaturverzeichnis hinter den Beispielen). So entwickeln 21-65% der akuten Schlaganfallpatienten Infektionen und 10-22% Pneumonien (Davenport et al. 1996, Castillo et al. 1998, Johnston et al. 1998, Grau et al. 1999, Georgilis et al. 1999, Langhorne et al. 2000). Im Vergleich mit der Inzidenz nosokomialer Infektionen, die bei durchschnittlich 7-10% aller Patienten auftreten (Bucher 2000) und unter postoperativen Patienten bei 3% liegen (Smyth & Emmerson 2000), wird die sehr hohe Infektionsrate bei akuten Schlaganfallpatienten besonders deutlich. In einer systematischen Untersuchung konnte aufgezeigt werden, dass am ersten und zweiten Tag nach dem Infarkt die Gefahr für eine Infektion am höchsten ist (Grau et al. 1999).

Die Verhinderung von Schlaganfall-induzierten Infektionen durch eine immunmodulatorische und insbesondere auch eine präventive antiinfektive Therapie stellt einen neuheitlichen Ansatz dar. Im Gegensatz zu der direkten neuroprotektiven Anwendung des Tetrazyklins Minocyclin (Yrjanheikki et al. 1998, 1999), die insbesondere einen unmittelbaren Einsatz nach dem Schlaganfall (bis 4 h nach dem Ereignis) erfordert, handelt es sich bei der beschriebenen Anwendung um ein verzögertes Behandlungsschema (12 - 72h), dass über die Verhinderung von schweren Schlaganfall-induzierten Infektionen zur Reduktion der Letalität und Morbidität führt. Tetrazykline sind darüber hinaus für die Verhinderung der typischerweise nach schwerem Schlaganfall auftretenden Infektionen ungeeignet.

Moxifloxacin gehört zur Klasse der Fluorochinolone und enthält den Wirkstoff *1-Cyclopropyl-6-fluor-1,4-dihydro-8-methoxy-7-[(4aS,7aS)-octahydro-6H-pyrrolo[3,4-b]-pyridin-6-yl]-4-oxochinolin-3-carbon-säure,* bislang vor allem eingesetzt zur Behandlung von Atemwegsinfektionen (Zhanel et al. 2002).

Mezlocillin gehört zur Klasse der Acylaminopenicilline und enthält den Wirkstoff *(2S, 5R, 6R)-3,3-Dimethyl-6-((R)-2-[3-(methylsulfonyl)-2-oxo-1-imidazolidincarboxamido]-2-phenylacetamideo)-7-oxo-4-thia-1-azabicyclo[3.2.0]heptan-2-carbonsäure.*

Sulbactam gehört zur Klasse der β-Laktamasehemmer und enthält den Wirkstoff *(2S,5R)-3,3-Dimethyl-7-oxo-4-thia-1-azabicyclo[3.2.0]heptan-2-carbonsäure-4,4-dioxid.*

Mezlocillin in Kombination mit Sulbactam wird bislang vor allem zur Behandlung von systemischen oder lokalen (Misch-) Infektionen sowie zur perioperativen Kurzzeitprophylaxe eingesetzt (Wright 1999).

Der Erfindung liegt die Aufgabe zugrunde, der medizinischen Praxis Mittel zur Verfügung zu stellen, die zur präventiven Therapie nach akutem Schlaganfall geeignet sind. Diese Aufgabe wird in einem ersten Aspekt durch den gezielten Einsatz von Antiinfektiva gelöst. Der Erfindung liegt ebenso die Aufgabe zugrunde, der medizinischen Praxis Mittel zur Verfügung zu stellen, die geeignet sind einer rasch nach einem akuten Schlaganfall einsetzenden Immundepression mit konsekutiver Infektionsanfälligkeit entgegenzuwirken.

Diese Aufgabe wird erfindungsgemäß durch den gezielten Einsatz immunmodulatorischer Substanzen, z.B. von Zytokinen, Inhibitoren des Sympathischen Nervensystems (SNS), inaktivierten Parapox-ovis-Viruspartikeln, Endotoxinbinder (rBPI21) gelöst. Es sind erfindungsgemäß Mittel zur immunmodulatorischen Therapie nach akutem Schlaganfall entwickelt worden, die als wirksame Komponenten bspw. Zytokine oder Inhibitoren des SNS, inaktivierten Parapox-ovis-Viruspartikeln, rBPI21 enthalten.

Es sind erfindungsgemäß Mittel zur präventiven Therapie nach akutem Schlaganfall entwikkelt worden, die als wirksame Komponenten Antiinfektiva und /oder immunmodulatorische Substanzen zur Prävention und Therapie von Pneumonien, Hamwegsinfektionen und Sepsis enthalten, vor allem aus den Klassen der Beta-Laktamantibiotika, Tetrazykline, Aminoglykoside, Linkosamine, Glykopeptide, Makrolide, Carbapeneme, Oxazolidinone, Streptogramine, sowie der Fluorochinolone - und hier insbesondere Moxifloxacin - sowie Zytokine (Interleukine, Interferone), Inhibitoren des Sympathischen Nervensystems (Beta-Blocker, Alpha-Sympathomimetika), Endotoxinbinder (Lipopolysaccharid-bindendes Protein = LBP, BPI, rBPI21) und inaktivierte Parapox-ovis-Viruspartikeln in pharmazeutischen Zubereitungen enthalten.

Es hat sich zudem herausgestellt, dass es vor allem durch das SNS vermittelt zu einer zellulär-funktionellen Immundepression nach Schlaganfall kommt, welche so schwer ausgeprägt sein kann, dass sie zur spontanen Entstehung schwerer, prognosebestimmender Infektionen führen kann. Die pharmakologische Hemmung des SNS, die Bindung von Endotoxinen, eine Immunstimulation oder die Gabe von Zytokinen (innerhalb der ersten 0 bis 7 Tage) kann die Immundepression aufheben und die Entstehung von Infektionen verhindern, weiterhin kann eine frühe präventive Antiinfektiva-Therapie (innerhalb der ersten 0 bis 7 Tage) zu einer Senkung der Letalität und Morbidität beim akuten Schlaganfall fuhren.

Erfindungsgemäß ist in einem allgemein anerkannten Tiermodell des Schlagfalls (Maus; Okklusion der A. cerebri media (MCAO) - Hata et al. 1998) eine früh einsetzende (innerhalb der ersten 12 Stunden) und lange anhaltende schwere Immunsuppression erkannt worden. Die Immunsuppression zeigt sich auf zellulärer Ebene einerseits in Form einer Lymphopenie der T-, B- und NK-Zellen sowie andererseits in Funktionsdefiziten der Monozyten und Lymphozyten unter anderem durch eine verminderte Sekretion des proinflammatorischen Zytokins IFN-γ. Die Immunsuppression ist vor allem durch eine Überaktivierung des SNS vermittelt.

Im Verlauf von 2 bis 4 Tagen nach dem Schlaganfall kommt es spontan zu einer schweren Infektion bei den Tieren. Es handelt sich hierbei um eine bakterielle Sepsis und Pneumonie, die bei ca. 60% der Tiere letal endet. Durch eine erfindungsgemäße frühe präventive Antiinfektivagabe (mit Moxifloxacin innerhalb der ersten 24 h) wird die Letalität und das neurologische Defizit drastisch gesenkt. In einem weiteren Aspekt der Erfindung konnte die Blockierung des SNS mit Betablockern oder durch die Gabe von IFN-γ die Entstehung der Infektionen ebenso verhindert werden. Zudem konnte durch eine frühe Blockade des SNS mit Hilfe eines Betablockers die Letalität nach akutem Schlaganfall drastisch gesenkt werden.

Die präventive Therapie mit Antiinfektiva (z.B. Moxifloxacin) innerhalb der ersten 7 Tage nach Erkrankungsbeginn stellt einen neuartigen Therapieansatz in der Behandlung von Schlaganfallpatienten dar, der geeignet ist, Letalität und Morbidität dieser Erkrankung zu senken. Insbesondere wird dadurch ein effektiver Therapieansatz für die Verhinderung der häufigen Infektiöns-Komplikationen zur Verfügung gestellt.

Diese Komplikationen führen zu einem verzögerten akut-stationären Verlauf und verhindern damit die notwendige und effektive Frührehabilitation. Sie bedingen zu einem großen Teil die relativ hohe Letalität nach akutem Schlaganfall (Henon et al. 1995, Katzan et al. 2003). Diese Komplikationen führen zu Fieber - einem unabhängigen Risikofaktor für einen ungünstigen Verlauf nach akutem Schlaganfall (Castillo et al. 1998). Die Behandlung dieser Komplikationen vermindert daher Letalität und Morbidität nach akutem Schlaganfall.

Patienten mit einem akuten Schlaganfall werden innerhalb von 72 Stunden nach Beginn der Symptomatik erfindungsgemäß mit einem Antibiotikum (Moxifloxacin) oder mehreren Antiinfektiva präventiv behandelt und so vor Infektionen (wie z.B. Pneumonien, Harnwegsinfektionen und Sepsis) geschützt. Damit wird die direkte und indirekte Letalität und Morbidität vermindert. Die präventive Antiinfektivatherapie soll über 1-7 Tage erfolgen.

Das Wesen der Erfindung liegt in der Verwendung bekannter Mittel zu einem neuen Zweck und in einer Kombination bekannter Elemente - den Antiinfektiva / Antibiotika - und einer neuen Wirkung - ihrem Einsatz zur Beeinflussung von Infektions-Komplikationen durch eine frühe präventive Antiinfektiva-Therapie nach akutem Schlaganfall - die in ihrer neuen Gesamtwirkung einen Vorteil und den erstrebten Erfolg ergeben, der darin liegt, dass nunmehr Mittel zur präventiven Therapie zur Verfügung stehen, die zu einer Senkung der Letalität und Morbidität, insbesondere auch zu einer Verbesserung der neurologischen Funktionen beim akuten Schlaganfall führen.

Die Erfindung richtet sich ferner auf die Verwendung von Antiinfektiva / Antibiotika zur präventiven antiinfektiven Therapie nach akutem Schlaganfall sowie zur Herstellung von Mitteln und / oder pharmazeutischen Zubereitungen zur präventiven antiinfektiven Therapie nach akutem Schlaganfall.

Dazu gehört ihre Verwendung zur Herstellung von Arzneimitteln zur präventiven Therapie von Pneumonien, Harnwegsinfektionen und Sepsis nach akutem Schlaganfall.

Unter einer frühen präventiven Antiinfektiva-Therapie nach akutem Schlaganfall wird verstanden, dass die Behandlung innerhalb von 72 Stunden nach dem Schlaganfall einsetzt.

Ein weiterer Aspekt der Erfindung stellt die immunmodulatorische Therapie mit Zytokinen (z.Bsp. IFN-γ), inaktivierten Parapockenviren (bspw. inaktivierten Parapoxvirus ovis), die Endotoxinbindung (bspw. mit rBPI21) oder die Blockade des SNS (z.B. Betablocker, z.B. Propranolol) oder die Aktivierung des Parasympathischen Nervensystems (z.B. CNI-1493) innerhalb der ersten 7 Tage nach Erkrankungsbeginn einen neuartigen Therapieansatz in der Behandlung von Schlaganfallpatienten dar, der geeignet ist, Letalität und Morbidität dieser Erkrankung zu senken. Insbesondere wird dadurch ein effektiver Therapieansatz für die Verhinderung der häufigen Infektions-Komplikationen zur Verfügung gestellt. Diese Komplikationen führen zu einem verzögerten akut-stationären Verlauf und verhindern damit die notwendige und effektive Frührehabilitation. Diese Komplikationen bedingen zu einem großen Teil die relativ hohe Letalität nach akutem Schlaganfall (Henon et al. 1995, Katzan et al. 2003). Diese Komplikationen bedingen des weiteren Fieber - einen unabhängigen Risikofaktor für einen ungünstigen Verlauf nach akutem Schlaganfall (Castillo et al. 1998). Die Verhinderung dieser Komplikationen vermindert daher Letalität und Morbidität nach akutem Schlaganfall. Insbesondere vermindert die immunmodulatorische Therapie dadurch des neurologische Defizit.

Patienten mit einem akuten Schlaganfall werden innerhalb von 72h Stunden nach Beginn der Symptomatik erfindungsgemäß mit einem Zytokin und/oder durch pharmakologische Sympathikusblockade und/oder durch pharmakologische Endotoxinbindung und/oder durch Aktivierung des Parasympathischen Nervensystems und/oder immunstimmulierend behandelt und so der Entstehung einer Immundepression vorgebeugt und vor Infektionen (wie z.B. Pneumonien, Harnwegsinfektionen und Sepsis) geschützt. Damit wird die direkte und indirekte Letalität und Morbidität vermindert. Diese immunmodulatorische Behandlung soll von Tag 0 - 7 nach akutem Schlaganfall erfolgen, je nach im Einzelfall durchzuführender klinischer Studie.

Das Wesen der Erfindung liegt in der Verwendung bekannter Mittel zu einem neuen Zweck und in einer Kombination bekannter Elemente - den Zytokinen, Immunstimulatien, Antisympatikotonika und Aktivatoren des Parasympathischen Nervensystems - und einer neuen Wirkung - ihrem Einsatz zur Verhinderung einer Immundepression nach akutem Schlaganfall - die in ihrer neuen Gesamtwirkung einen Vorteil und den erstrebten Erfolg ergeben, der darin liegt, dass nunmehr Mittel zur präventiven Therapie zur Verfügung stehen, die zu einer Senkung der Letalität und Morbidität beim akuten Schlaganfall führen.

Die Erfindung richtet sich ferner auf die Verwendung von Zytokinen und Pharmaka zur Blokkade des SNS, Endotoxinbindem und Immunstimulantien zur immunmodulatorischen Therapie nach akutem Schlaganfall sowie zur Herstellung von Mitteln und / oder pharmazeutischen Zubereitungen zur immunmodulatorischen Therapie nach akutem Schlaganfall.

Dazu gehört ihre Verwendung zur Herstellung von Arzneimitteln zur Linderung oder Aufhebung einer Immundepression nach akutem Schlaganfall. Unter einer frühen immunmodulatorischen Therapie nach akutem Schlaganfall wird verstanden, dass die Behandlung innerhalb von 1 Woche nach dem Schlaganfall einsetzt.

### Detaillierte Beschreibung der Erfindung

Die Erfindung betrifft die Verwendung eines Antiinfektivums und/oder Immunmodulators zur Herstellung eines Mittels und oder einer pharmazeutischen Zubereitungen zur präventiven antiinfektiven Therapie nach akutem Schlaganfall. Es sind erfindungsgemäß Mittel und pharmazeutische Zubereitungen zur präventiven Therapie nach akutem Schlaganfall entwikkelt worden, die als wirksame Komponenten Antiinfektiva zur Prävention und Therapie von Pneumonien, Harnwegsinfektionen und Sepsis enthalten, vor allem aus den Klassen der Beta-Laktamantibiotika, Tetrazykline, Aminoglykoside, Linkosamine, Glykopeptide, Makrolide, Carbapeneme, Oxazolidinone, Streptogramine, sowie der Fluorochinolone - und hier insbesondere Moxifloxacin - sowie Zytokine (Interleukine, Interferone), Inhibitoren des Sympathischen Nervensystems (Beta-Blocker, Alpha-Sympathomimetika), Aktivatoren des Parasympathischen Nervensystems, Endotoxinbinder (Lipopolysaccharid-bindendes Protein = LBP, BPI, rBPI21) und inaktivierte Parapox-ovis-Viruspartikeln in pharmazeutischen Zubereitungen enthalten.

Die präventive Therapie mit Antiinfektiva (z.B. Moxifloxacin) innerhalb der ersten 7 Tage nach Erkrankungsbeginn stellt einen neuartigen Therapieansatz in der Behandlung von Schlaganfallpatienten dar, der geeignet ist, Letalität und Morbidität dieser Erkrankung zu senken.

Die häufig auftretenden Infektionskomplikationen führen zu einem verzögerten akut-stationären Verlauf und verhindern damit die notwendige und effektive Frührehabilitation. Zudem bedingen sie zu einem großen Teil die relativ hohe Letalität nach akutem Schlaganfall (Henon et al. 1995, Katzan et al. 2003)) und verursachen des weiteren Fieber, das als unabhängiger Risikofaktor einen ungünstigen Verlauf nach akutem Schlaganfall veruracht (Castillo et al. 1998). Die Behandlung dieser Komplikationen vermindert daher Letalität und Morbidität nach akutem Schlaganfall.

Patienten mit einem akuten Schlaganfall werden zum frühesten möglichen Zeitpunkt spätestens jedoch innerhalb von 72 Stunden nach Beginn der Symptomatik erfindungsgemäß mit einem Antiinfektivum, insbesondere einem Breitbandantibiotikum, insbesondere einem Antibiotikum (Moxifloxacin) oder mehreren Antiinfektiva behandelt. Damit wird die direkte und indirekte Letalität und Morbidität vermindert. Die präventive Antiinfektivatherapie soll über 1-7 Tage erfolgen. Die bevorzugte Dosierung richtet sich nach der jeweils üblichen wirksamen Tagesdosis des/der Antiinfektiva zur Behandlung der o.g. Infektionen. Das Antiinfektivum wird bevorzugterweise oral oder parenteral verabreicht. Moxifloxacin wird in der Dosierung 400 mg einmal am Tag oral oder parenteral verabreicht.

Das zur Anwendung kommende Antiinfektivum kann ein oder mehrere Antibiotika in pharmazeutischer Zubereitung enthalten. In einer besonderen Ausführungsform der Erfindung werden mehrere verschieden Antiinfektiva kombiniert.

Bevorzugt werden die Antibiotika aus den Klassen der Beta-Laktamantibiotika, Tetrazykline, Aminoglykoside, Linkosamine, Glykopeptide, Makrolide, Carbapeneme, Oxazolidinone, Streptogramine, sowie der Fluorochinolone.. Hiervon werden besonders bevorzugt Breitbandantibiotika, besonders Betalaktamantibiotika, Fluorochinolone und Carbapeneme. Am bevorzugtesten kommt Moxifloxacin *(1-Cyclopropyl-6-fluor-1,4-dihydro-8-methoxy-7-[(4aS, 7aS)-octahydro-6H-pyrrolo[3,4-b]-pyridin-6-yl]-4-oxochinolin-3-carbon-säure*) zur Verwendung da Moxifloxacin die beim Schlaganfall-Patienten auftretenden Infektionserreger erfasst und nur einmal pro Tag appliziert werden muss.

Die erfindungsgemäße Verwendung eines Antiinfektivums zur Herstellung von Mitteln und / oder pharmazeutischen Zubereitungen zur präventiven antiinfektiven Therapie nach akutem Schlaganfall ist für Säugetiere, besonders bei Haus- und Nutztieren, insbesondere für den Menschen nutzbringend. Nebst der Hauptanwendung im humanen Bereich können demgemäß gleichermaßen vetereninärmedizinische Produkte bzw. Medikamente hergestellt werden.

Bevorzugt wird die Verwendung eines Antiinfektivums zum Schutz vor Infektionen - Pneumonien, Harnwegsinfektionen und / oder Sepsis - nach akutem Schlaganfall.

Gemäß der vorliegenden Erfindung kommt ein Immunmodulator zur Herstellung einer pharmazeutischen Formulierung zur Anwendung der dadurch gekennzeichnet ist, dass dieser ausgewählt sein kann aus der Gruppe, welche Zytokine und / oder Inhibitoren des SNS und / oder Aktivatoren des Parasympathischen Nervensystems umfassen. Diese haben die Fähigkeit eine Immundepression, insbesondere den sogenannten Th1/Th2 Shift zu verhindern oder zu kompensieren. Bevorzugt werden Interferone, Interleukine und Betarezeptorenblocker angewendet werden. Am bevorzugtesten sind der β-Blocker Propranolol, das Interferon IFN-γ, der Endotoxinbinder rBPI-21, Lipopolysaccharid-bindendes Protein = LBP, sowie inaktivierte Parapox-ovis-Viruspartikel.

Der oder die Immunmodulatoren werden zur Herstellung eines Medikamentes bzw. pharmazeutischen Formulierung gemäß der Erfindung verwendet die zur präventiven antiinfektiven Therapie nach akutem Schlaganfall bei Säugetieren, besonders bei Haus- und Nutztieren, insbesondere beim Menschen dienen kann.

Die Erfindung sieht auch die Verwendung eines oder mehrerer Antiinfektiva und eines oder mehrerer Immunmodulatoren zur Herstellung einer pharmazeutischen Zubereitungen zur präventiven, antiinfektiven Therapie nach akutem Schlaganfall vor wobei das Antiinfektivum ausgewählt sein kann aus der Gruppe welche Breitbandantibiotika umfasst und der Immunmodulator ausgewählt ist aus der Gruppe welche Inhibitoren des SNS, Zytokine, Endotoxinbinder und inaktivierte Parapox-ovis-Viruspartikeln umfasst. Bevorzugt ist das Antiinfektivum Moxifloxacin und der Immunmodulator IFN-γ.

Die Erfindung beschreibt auch ein Kit, dieser umfasst in getrennter oder kombinierter Form eine Pharmazeutische Zusammensetzung enthaltend einen Immunmodulator welcher ausgewählt sein kann, aus der Gruppe der Zytokine und / oder Inhibitoren des SNS und ein Antiinfektivum welcher ausgewählt sein kann, aus der Gruppe der Beta-Laktamantibiotika, Tetrazykline, Aminoglykoside, Linkosamine, Glykopeptide, Makrolide, Carbapeneme, Oxazolidinone, Streptogramine, sowie der Fluorochinolone.

Die Erfindung beschreibt weiterhin ein Verfahren zur Behandlung von Patienten nach akutem Schlaganfall, mit dem Ziel, die Letaliät zu senken, wobei auch Infektionen nach dem Schlaganfall verhindert werden sollen. Hierbei wird einem Patienten welcher diese Behandlung benötigt, also einen Schlaganfall erlitten hat eine wirksame Dosis eines Antiinfektivums und/oder eines Immunmodulators verabreicht. Bevorzugt wird dem Patienten die Dosis direkt nach dem Schlaganfall verabreicht. Es können auch Kombinationen aus einem oder mehrerer Antiinfektiva verabreicht werden und einem oder mehrer Immunmodulatoren. Bevorzugt werden folgende Kombinationen aus einem oder mehreren Breitbandantibiotika sowie aus einem oder mehreren Zytokinen und /oder Endotoxinbindem und/oder Parapoxwis-Viruspartikeln. An den folgenden 1-7 Tagen sollen die jeweils üblichen Tagesdosen der o.g. Antiinfektiva (1mg- 100g) und Immunmodulatoren (1µg-100g) verwendet werden.

Die erfindungsgemäße Verwendung der Mittel kann durch bekannte Formulierungen geschehen, also Formulierungen, die *bis dato* für die jeweiligen Wirkstoffe zur Anwendung kam. Die Erfindung sieht aber auch vor, neue Formulierungen zu erstellen, die speziell für die Infarktpatienten geeignet sind, z.B. für intrathekale, nasale, intramuskuläre, intratracheale, intrabronchiale, subcutane, intravenöse, intraarterielle, permucosale, enterale und orale.

Die erfindungsgemäße Verwendung der Mittel kann in bekannter Weise in die üblichen Formulierungen überführt werden, wie Tabletten, Dragees, Pillen, Granulate, Aerolsole, Pillen, Emulsionen, Suspensionen und Lösungen, unter Verwendung inerter, nicht toxischer, pharmazeutisch geeigneter Trägerstoffe, oder Lösungsmittel. Hierbei soll die therapeutisch wirksame Mittelkonzentration jeweils in einer Konzentration vorliegen wie sie für diese Mittel üblicherweise bisher angewendet wurde, bzw. von etwa 0,1 bis 95 Gew.-%, bevorzugt von etwa 0,5 bis 90 Gew.-% der Gesamtmischung vorhanden sein, d.h. in Mengen" die ausreichend sind, um den angegebenen Dosierungsspielraum zu erreichen. Die Formulierungen werden beispielsweise hergestellt durch Verstrecken der Mittel mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfsstoffe verwendet werden können.

Als Hilfsstoffe erwähnt seien z.B. Wasser, nicht-toxische Lösungsmittel, wie Parafin, (z.B. Erdölfraktionen), Pflanzliche Öle (z.B. Erdnuß/-Sesamöl), Alkohole, (z.B. Ethylalkohol, Glycerin), Trägerstoffe, wie z.B. natürliche Gesteinsmehle (z.B. Kaoline, Tonerde, Talkum, Kreide) synthetische Gesteinsmehle (z.B. hochdisperse Kieselsäure, Silikate), Zucker (z.B. Rohr-, Milch- und Traubenzucker) , Emulgiermittel (z.B. Polyoxyethylen-Fettsäure-Ester, Polyoxyethylen-Fettalkohol-Ether), Dispergiermittel (z.B. Lignin, Sulfitablaugen. Methylcellulose, Stärke und Polyvinylpyrolidon und Gleitmittel (z.B. Magnesiumstearat, Talkum, Stearinsäure, und Natriumsulfat). Die Applikation erfolgt in üblicher weise, bevorzugt oral oder parenteral, insbesondere perlingual oder intravenös. Im Falle der oralen Anwendung der erfindungsgemäßen Medikamente können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine, und dergleichen enthalten. Weiterhin können Gleitmittel, wie Magnesiumstearat, Natriumlaurylsulfat und Talkum zum Tablettieren mitverwendet werden. Im Falle wässriger Suspensionen können die Wirkstoffe außer den oben genannten Hilfsstoffen mit verschiedenen Geschmacksaufbesserem oder Farbstoffen versetzt werden. Für den Fall der parenteralen Anwendung können Lösungen der Wirkstoffe unter Verwendung geeigneter flüssiger Trägermaterialien eingesetzt werden.

Die Erfindung umfasst ferner einen Kit umfassend in getrennter Form eine Pharmazeutische Zusammensetzung enthaltend einen Immunmodulator welcher ausgewählt sein kann, aus der obig beschriebenen Gruppe der Immunmodulatoren und ein Antiinfektivum welches ausgewählt sein kann, aus der obig beschriebenen Gruppe der Antiinfektiva.

Die Erfindung umfasst ferner die Verwendung von Immunmodulatoren und Antiinfektiva zur Herstellung einer pharmazeutischen Zusammensetzung bzw. Medikamentes, welcher beide Stoffe enthält. Gleichermaßen können von beiden Wirkungsgruppen mehrere vorhanden sein. Bevorzugt sind Breitbandantibiotika sowie Inhibitoren des SNS und / oder Aktivatoren des Parasympathischen Nervensystems und / oder Zytokinen und / oder Endotoxinbindem und / oder Parapox-ovis-Vinlspartikeln.

Bevorzugtere Kombinationen sind Beta-Laktamantibiotika, Fluorochinolone und Carbapeneme und / oder Zytokinen. Am Bevorzugtesten sind Moxifloxacin IFN-γ.

Die Erfindung soll anhand von Ausführungsbeispielen näher erläutert werden, ohne auf diese Beispiele beschränkt zu sein.

### Ausführungsbeispiele Antiinfektivum

### Beispiel 1 A: Mausmodell der zerebralen Ischämie

Nach dem Mausmodell der zerebralen Ischämie, einem generell akzeptiertem Modell des ischämischen Schlaganfalls, wurden Mäuse MCAO-operiert. Dabei wird die A.cerebri media für ca. 60 min verschlossen und es entsteht ein für das Modell typischer zerebraler Infarkt. Als Kontrolle dient die sogenannte Sham-Operation, bei der die Tiere gleichfalls MCAO-operiert werden, die A.cerebri media jedoch nur für ca. 1 min verschlossen wird. Ansonsten sind beide Paradigmen identisch. Damit kann der perioperative Stress als systematischer Fehler für die folgenden Ergebnisse als sicher ausgeschlossen gelten.

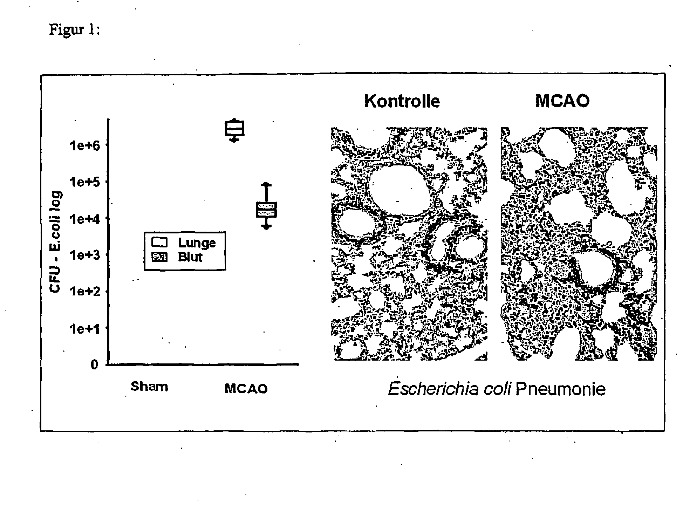

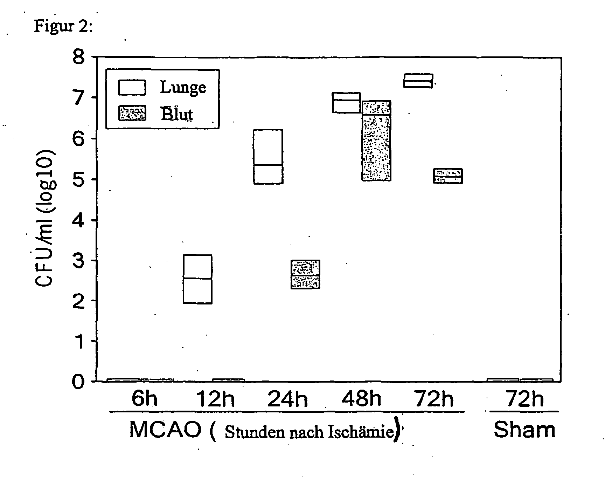

Figur 1 zeigt, dass die Mäuse 3 Tage nach Schlaganfall eine Bakteriämie und eine Pneumonie mit einer bakteriellen Infektionslast (mehr als 95% Escherichia coli) von 2*10⁴ bzw. 4*10⁶ Kolonie-formenden Einheiten/ml (CFU/ml) haben. Im Gegensatz dazu wird keine Infektion in den Kontrolltieren (Sham-operiert) gefunden. Figur 2 zeigt den typischen Zeitverlauf für die Schlaganfall-induzierten spontanen bakteriellen Infektionen. Eine signifikante bakterielle Infektionslast wird ca. 24 Stunden nach dem Schlaganfall beobachtet.

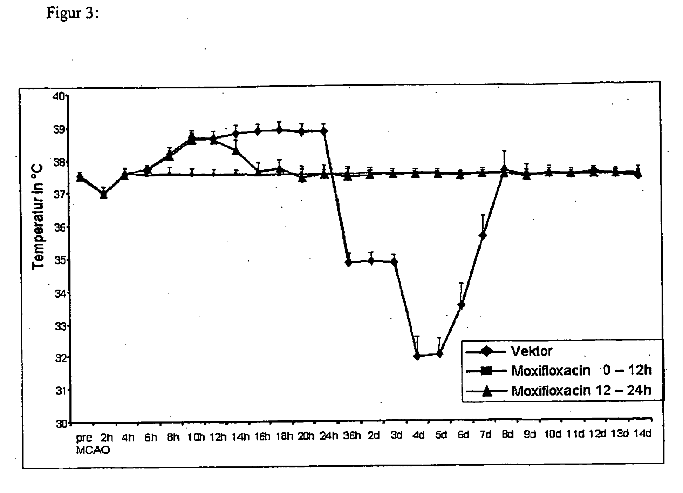

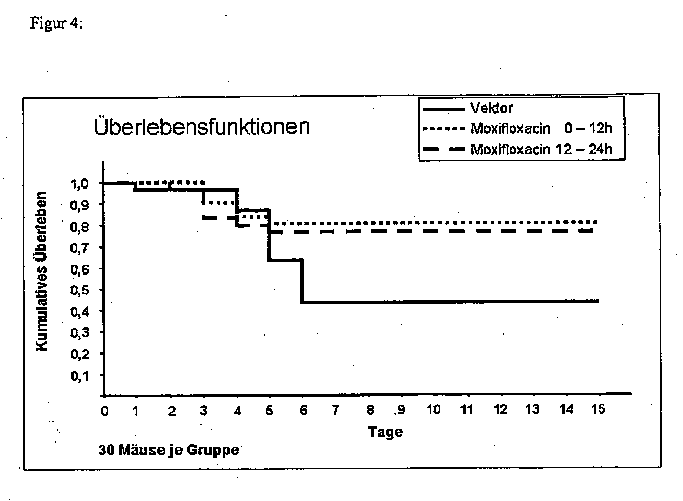

Die Schlaganfalltiere entwickeln innerhalb der ersten 12 Stunden (h) nach dem Schlaganfall Fieber und beginnen nach weiteren 12 h eine hypotherme Körpertemperatur zu bekommen (Figur 3, Vektor-Gruppe). Im weiteren Verlauf entwickelt sich aus der Bakteriämie/Pneumonie das Bild einer Sepsis, an der die Tiere nach 4-6 Tagen versterben (Figur 4, Vektor-Gruppe).

### Beispiel 2A: Einsatz eines Antiinfektivums

Durch eine präventive Therapie mit Hilfe des Antiinfektivums Mezlocillin plus Sulbactam wurden mittels eines sehr frühen - 0 bis 12 h nach akutem Schlaganfall - Behandlungsschemas sowohl die Infektionen (Daten nicht gezeigt) verhindert als auch Fieber/Hyperthermie und insbesondere Letalität nach Schlaganfall signifikant reduziert.

### Beispiel 3A: Einsatz von Mezlocillin plus Sulbactam

Durch eine präventive Therapie in Anlehnung an Beispiel 2 unter Einsatz von Mezlocillin plus Sulbactam wurden mittels eines späteren - 12 bis 24 h nach akutem Schlaganfall - Behandlungsschemas sowohl die Infektionen verhindert (Daten nicht gezeigt) als auch Fieber/Hyperthermie und insbesondere Letalität nach Schlaganfall signifikant reduziert.

### Beispiel 4A: Einsatz von Imipenem plus Cilastatin

Durch eine präventive Therapie in Anlehnung an Beispiel 2 unter Einsatz von Imipenem plus Cilastatin wurden mittels eines sehr frühen - 0 bis 16 h nach akutem Schlaganfall - Behandlungsschemas sowohl die Infektionen verhindert (Daten nicht gezeigt) als auch Fieber/Hyperthermie und insbesondere Letalität nach Schlaganfall signifikant reduziert.

### Beispiel 5A: Einsatz von Moxifloxacin

Durch eine präventive Therapie mit Moxifloxacin wurden durch ein sehr frühes (0-12 h nach akutem Schlaganfall) als auch in einem etwas späteren (12-24 h nach akutem Schlaganfall) Behandlungsschema sowohl die Infektionen (Pneumonie, Sepsis) verhindert als auch Fieber/Hyperthermie, Letalität und insbesondere auch das neurologische Defizit nach Schlaganfall signifikant reduziert (Figuren 3 bis 5).

### Ausführungsbeispiele Immunmodulation

### Beispiel 1B: Mausmodell der zerebralen Ischämie

Nach dem Mausmodell der zerebralen Ischämie wurden Mäuse MCAO-operiert, wobei die A.cerebri media für ca. 60 min verschlossen wird und ein für das Modell typischer zerebraler Infarkt entsteht. Als Kontrolle dient die sogenannte Sham-Operation, bei der die Tiere gleichfalls MCAO-operiert werden, die A.cerebri media jedoch nur für ca. 1 min verschlossen wird. Ansonsten sind beide Paradigmen identisch. Damit kann der perioperative Stress als systematischer Fehler für die folgenden Ergebnisse als sicher ausgeschlossen gelten.

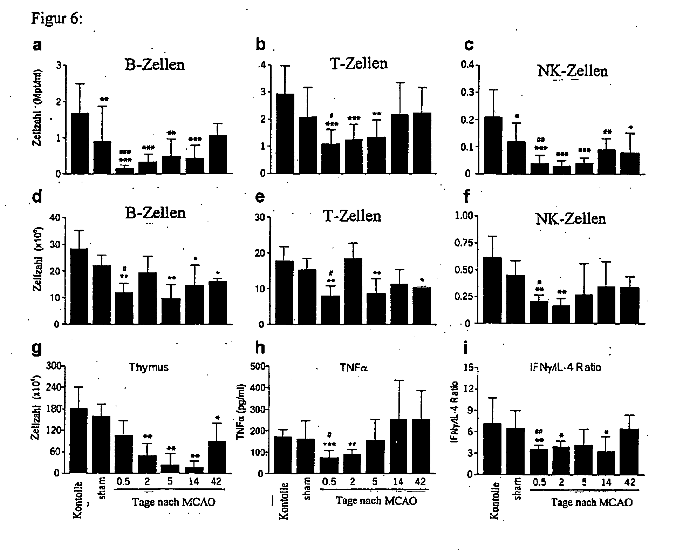

Figur 6 zeigt, dass die Mäuse innerhalb eines halben Tages nach dem Schlaganfall eine deutliche Reduktion in der Zahl ihrer im Blut zirkulierenden Lymphozyten (im Detail: B-Zellen, T-Zellen und NK-Zellen) erfahren (Fig 6:a-c). Diese schwere Lymphopenie hält mindestens 14 Tage lang an und betrifft auch die Milz und den Thymus (Fig 6: d - g), welche wesentliche immunologische Reifungsorgane sind. Neben der rein numerischen Abnahme findet sich auch eine funktionelle Beeinträchtigung mit Störung der Zytokinsektretionskapazität nach Stimulation (Fig 6: h und j). Neben der Monozytenfunktionsstörung, welche sich in einer TNF-α-Sekretionsstörung für 2 Tage nach LPS-Stimulation ausdrückt, ist eine Lymphozytenalteration nach ConA-Stimulation nachweisbar, die 14 Tage nach dem Schlaganfall anhält und im Wesentlichen einen Th1 - zu Th2 - Shift bedingt (veränderte IFN-γ / IL4 - Ratio), was einen Verlust an proinflammatorischer Potenz bedeutet. Auch die Sham-operierten Tiere weisen Veränderungen vorgenannter Parameter auf, jedoch sind diese kleiner. B- und NK-Zellen fallen nach dem OP-Streß etwas nur im Blut ab. In der Milz und im Thymus lassen sich stress-bedingt keine wesentlichen Veränderungen ausmachen und die funktionellen Parameter der Monozyten und Lymphozyten bleiben auf Kontrollniveau. Figur 1 und 2 zeigen, dass die Mäuse 3 Tage nach Schlaganfall eine Bakteriämie und eine Pneumonie mit einer bakteriellen Infektionslast (mehr als 95% Escherichia coli) von 2*10⁵ bzw. 4*10⁷ Kolonie-formenden Einheiten/ml (CFU/ml) haben. Im Gegensatz dazu wird keine Infektion in den Kontrolltieren (Sham-operiert) gefunden.

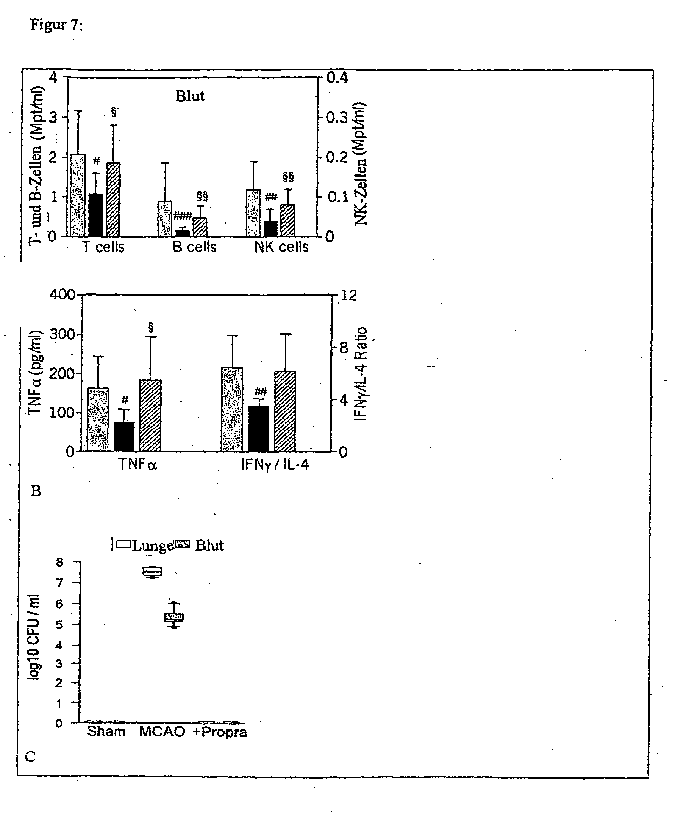

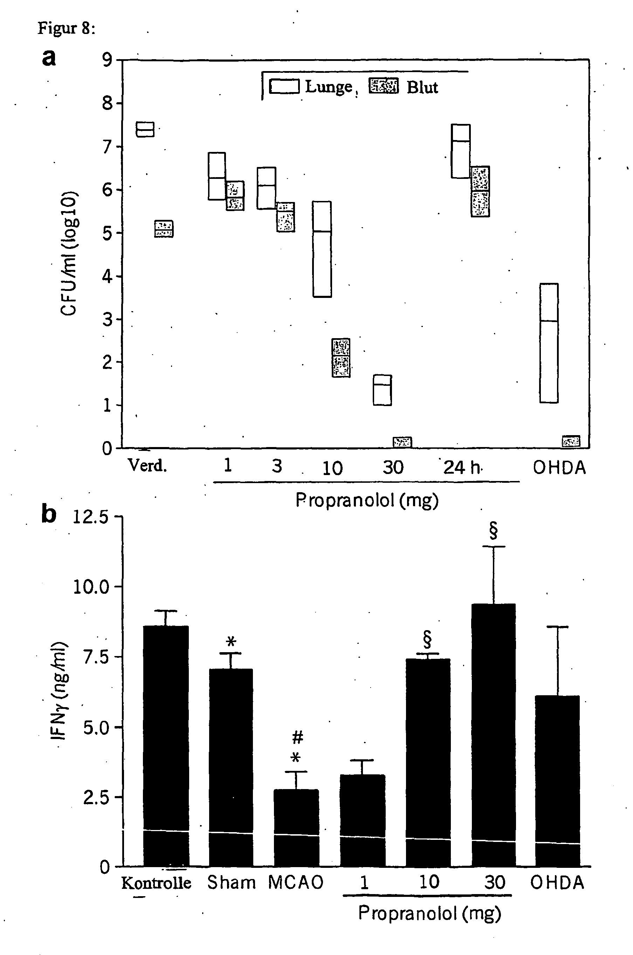

Beispiel 2B: Blockade des SNS durch Gabe von Propranolol (einem unspezifischen Betablocker) nach experimentellem Maus - Schlaganfall konnte der Abfall der T- und NK - Zellen verhindert werden und das Ausmaß der B-Zelllymphopenie deutlich reduziert werden (Fig 7). Außer dem verhindert die Blockade des SNS durch Propranolol (30mg/kg Körpergewicht) auch die Funktionsstörung der verbleibenden Monozyten (TNF-α- Sekretion) und Lymphozyten (IFN-γ / IL4 - Ratio) (Fig 7). Die Blockade des SNS kann ebenso die Entstehung der Infektionen in Lunge (Pneumonie) und Blut (Sepsis) verhindern (Fig 7). Die Propranololdosis (Angaben in mg/kg Körpergewicht), die zur Verhinderung bzw. drastischen Reduktion der bakteriellen Infektionen notwendig ist, verhindert auch die gestörte INF-γ Sekretion der Lymphozyten (Figur 8). Dabei ist die Gabe von Propranolol sehr frühzeitig (unmittelbar bis 12 Stunden) nach dem Schlaganfall notwendig. Eine Gabe 24 Stunden nach dem Ereignis bleibt ohne Wirkung (Figur 8). Eine frühe (vor dem Schlaganfall) chemische Sympathikolyse durch 6-Hydroxydopamin (6-OHDA) verhindert ebenfalls die Schlaganfall-induzierte gestörte INFy Funktion der Lymphozyten und blockt die schweren bakteriellen Infektionen (Figur 8).

### Beispiel 3B: Gabe von Zytokinen

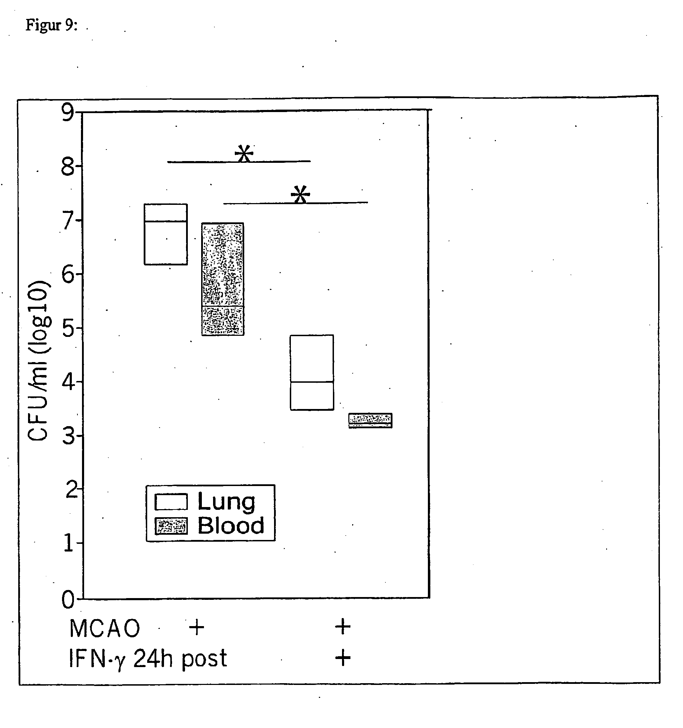

Durch die Gabe von IFN-γ (2µg) konnte sowohl die Keimzahl in der Lunge als auch noch deutlicher die Keimzahl im Blut reduziert werden (Figur 9).

### Beispiel 4B: Gabe eines β-Blockers

Durch die Gabe von Propranolol (30mg/kg Körpergewicht) konnten nicht nur die Infektionen verhindert werden (Figuren 7 und 8) sondern auch das Überleben nach Schlaganfall drastisch verbessert werden (Figur 10).

### Legende zu den Figuren

Figur 1: Bakteriämie und Pneumonie 3 Tage nach experimentellem Schlaganfall
Figur 2: Früher Verlauf von Bakteriämie und Pneumonie nach experimentellem Schlaganfall
Figur 1B: Zelldifferenzierung mittels FACS-Analyse aus Blut (a-c), Milz (d-f) und Thymus (g) und Lymphozytenfunktionstests von Monozyten (h) und T-Zellen (i).
Figur 3: Hyper- und Hypothermie nach experimentellem Schlaganfall und deren effektive Verhinderung durch eine frühe präventive Therapie mittels Antiinfektivum.
Figur 4: Letalität nach experimentellem Schlaganfall und deren effektive Verhinderung durch eine frühe präventive Therapie mittels eines Antiinfektivums

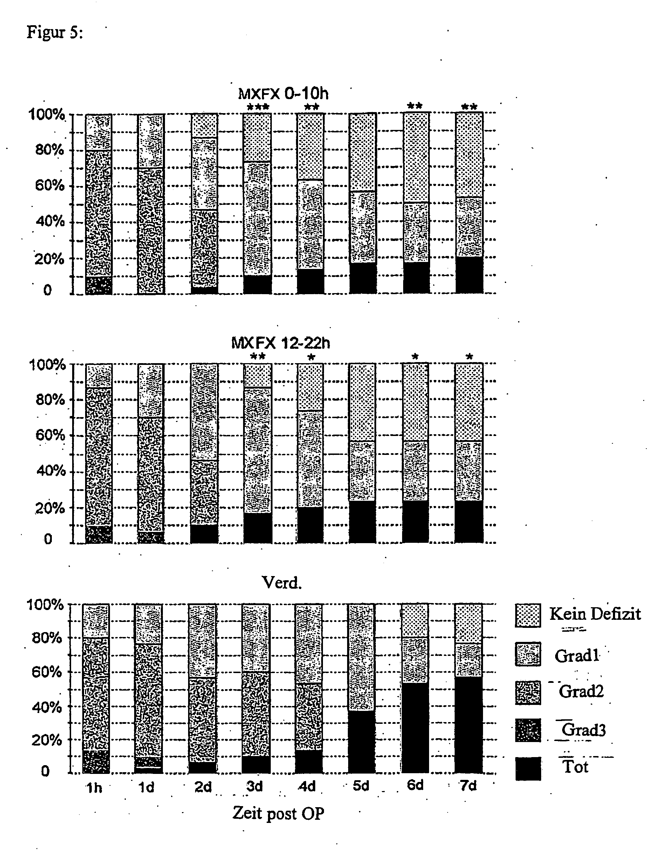

Figur 5: Neurologisches Defizit nach experimentellem Schlaganfall und dessen effektive Verhinderung durch eine frühe präventive Therapie mittels eines Antiinfektivums
Figur 6: Zelldifferenzierung mittels FACS-Analyse aus Blut (a-c), Milz (d-f) und Thymus (g) und Lymphozytenfunktionstests von Monozyten (h) und T-Zellen (i).
Figur 7: Verhinderung der Lymphopenie (a), Lymphozytenfunktionsstörung (b) und Entstehung schwerer Infektionen (c) durch pharmakologische Sympathikusblockade.
Figur 8: Dosis- und Zeit-abhängige Verhinderung der Entstehung schwerer bakterieller Infektionen (a) und der Lymphozytenfunktionsstörung (b) durch pharmakologische Sympathikusblockade.
Figur 9: Attenuierung der Infektionsschwere in Lunge und Blut (Zahl der CFU) durch Gabe eines Zytokins (INF-γ).

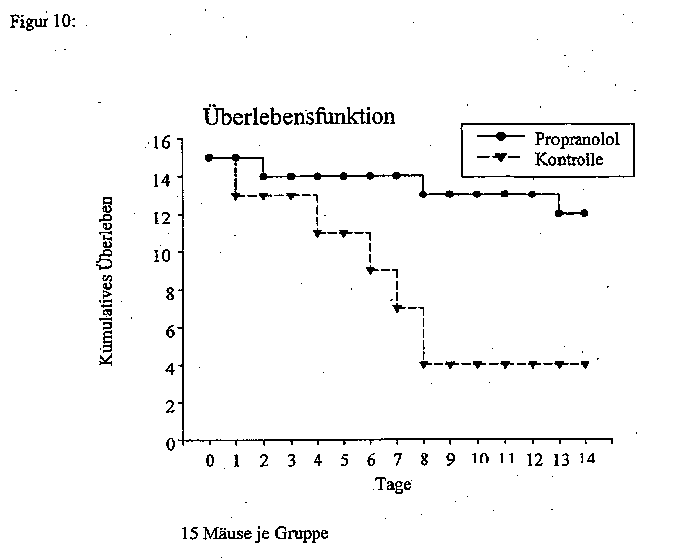

Figur 10: Letalität nach experimentellem Schlaganfall und deren effektive Verhinderung durch eine frühe Immunmodulation mittels eines β-Blockers.

### Literaturverzeichnis

Bucher A (2000). Hand hygiene-is hand disinfection the best solution. Tidsskr Nor Laegeforen 120:472-5
Castillo J, Dávalos A, Marrugat J, Noya M (1998). Timing for fever-related brain damage in acute ischemic stroke. Stroke 29:2455-2460.
Davenport RJ, Dennis MS, Wellwood I, Warlow CP (1996). Complications after acute stroke. Stroke 27:415-20
Georgilis K, Plomaritoglou A, Dafni U, Bassiakos Y, Vemmos K (1999). Aetiology of fever in patients with acute stroke. J Intern Med 246:203-9
Grau AJ, Buggle F, Schnitzler P, Spiel M, Lichy C, Hacke W (1999). Fever and infection early after ischemic stroke. J Neurol Sci 171:115-20
Hata R, Mies G, Wiessner C, Fritze K, Hesselbarth D, Brinker G, Hossmann KA (1998). A reproducible model of middle cerebral artery occlusion in mice: hemodynamic, biochemical, and magnetic resonance imaging. J Cereb Blood Flow Metab 18:367-75
Henon H, Godefroy O, Leys D, Mounier-Vehier F, Lucas C, Rondepierre P, Duhamel A, Pruvo JP (1995). Early predictors of death and disability after acute cerebral ischemic event. Stroke 26:392-8
Johnston KC, Li JY, Lyden PD, Hanson SK, Feasby TE, Adams RJ, Faught RE Jr, Haley EC Jr (1998). Medical and neurological complications of ischemic stroke: experience from the RANTTAS trial. RANTTAS Investigators. Stroke 29:447-53
Katzan IL, Cebul RD, Husak SH, Dawson NV, Baker DW (2003). The effect of pneumonia on mortality among patients hospitalized for acute stroke. Neurology 60:620-5
Langhorne P, Stott DJ, Robertson L, MacDonald J, Jones L, McAlpine C, Dick F, Taylor GS, Murray G (2000). Medical complications after stroke: a multicenter study. Stroke 31:1223-9
Smyth ET, Emmerson AM (2000). Surgical site infection surveillance. J Hosp Infect 45:173-84
Wright AJ (1999).The penicillins. Mayo Clin Proc 74:290-307
Yrjanheikki J, Keinanen R, Pellikka M, Hokfelt T, Koistinaho J. (1998). Tetracyclines inhibit microglial activation and are neuroprotective in global brain ischemia. Proc Natl Acad Sci U S A 95:15769-74
Yrjanheikki J, Tikka T, Keinanen R, Goldsteins G, Chan PH, Koistinaho J. (1999). A tetracycline derivative, minocycline, reduces inflammation and protects against focal cerebral ischemia with a wide therapeutic window. Proc Natl Acad Sci U S A 96:13496-500.
Zhanel GG, Ennis K, Vercaigne L, Walkty A, Gin AS, Embil J, Smith H, Hoban DJ (2002). A critical review of the fluoroquinolones: focus on respiratory infections. Drugs 62:13-59

## Patentansprüche

1. Verwendung eines oder mehrerer Antiinfektiva und/oder eines oder mehrerer Immunmodulatoren zur Herstellung eines Mittels und/oder einer pharmazeutischen Zubereitung zur präventiven antiinfektiven Therapie nach akutem Schlaganfall, wobei mit der Therapie 12 bis 72 Stunden nach dem Schlaganfall begonnen wird.

2. Verwendung eines Antiinfektivums nach Anspruch 1 **dadurch gekennzeichnet, daß** das Antiinfektivum ein oder mehrere Antibiotika in pharmazeutischer Zubereitung enthält.

3. Verwendung eines Antiinfektivums nach Anspruch 2, wobei das oder die Antibiotika aus den Klassen der Beta-Laktamantibiotika, Tetrazykline, Aminoglykoside, Linkosamine, Glykopeptide, Makrolide, Carbapeneme, Oxazolidinone, Streptogramine und / oder der Fluorochinolone ausgewählt sein können.

4. Verwendung eines Antiinfektivums nach Anspruch 2 oder 3, wobei das oder die Antibiotika aus den Klassen der Beta-Laktamantibiotika, Carbapeneme, und/oder der Fluorochinolone ausgewählt sein können.

5. Verwendung eines Antiinfektivums nach einem der Ansprüche 1 bis 4, **dadurch gekennzeichnet, daß** Moxifloxacin *(1-Cyclopropyl-6-fluor-1,4-dihydro-8-methoxy-7-[(4aS,7aS)-octahydro-6H-pyrrolo[3,4-b]-pyridin-6-yl]-4-oxochinolin-3-carbon-säure)* enthalten ist.

6. Verwendung eines Antiinfektivums nach Anspruch 3 oder 4 zur Herstellung einer pharmazeutischen Zubereitung, wobei Mezlocillin und Sulbactam zusammen verwendet werden.

7. Verwendung eines Antiinfektivums nach einem der Ansprüche 1 bis 6 zur Herstellung von Mitteln und / oder pharmazeutischen Zubereitungen zur präventiven antiinfektiven Therapie nach akutem Schlaganfall bei Säugetieren, besonders bei Haus- und Nutztieren, insbesondere beim Menschen.

8. Verwendung eines Antiinfektivums nach einem der Ansprüche 1 bis 7 zum Schutz vor Infektionen, wie Pneumonien, Hamwegsinfektionen und / oder Sepsis, nach akutem Schlaganfall.

9. Verwendung eines Immunmodulators nach Anspruch 1, **dadurch gekennzeichnet, dass** der Immunmodulator ausgewählt ist aus der Gruppe, welche Zytokine und / oder Inhibitoren des SNS oder Endotoxinbinder oder Parapox-ovis-Viruspartikel umfassen.

10. Verwendung nach Anspruch 8 wobei Propranolol und/oder IFN-γ zur Anwendung kommt(en).

11. Verwendung nach einem der Ansprüche 7 bis 9 zur Herstellung von Mitteln und / oder pharmazeutischen Zubereitungen zur präventiven antiinfektiven Therapie nach akutem Schlaganfall bei Säugetieren, besonders bei Haus- und Nutztieren, insbesondere beim Menschen.

12. Verwendung eines oder mehrerer Antiinfektiva und eines oder mehrerer Immunmodulatoren zur Herstellung einer pharmazeutischen Zubereitung zur präventiven, antiinfektiven Therapie nach akutem Schlaganfall nach einem der obigen Ansprüche wobei das Antiinfektivum ausgewählt sein kann aus der Klasse welche Betalaktamantibiotika, Tetrazykline, Aminoglykoside, Linkosamine, Glykopeptide, Makrolide, Carbapeneme, Oxazolidinone, Streptogramine sowie der Fluorochinolone umfasst und der Immunmodulator ausgewählt ist aus der Gruppe welche Zytokine (Interleukine, Interferone), Inhibitoren des Sympathischen Nervensystems (Beta-Blocker, Alpha-Sympathomimetika), Endotoxinbinder und inaktivierte Parapox-ovis-Viruspartikeln umfasst.

13. Verwendung nach Anspruch 12, wobei das Antiinfektivum Moxifloxacin ist und der Immunmodulator IFN-γ ist.

## Claims

1. Use of one or more anti-infective agents and/or one or more immunomodulators for producing an agent and/or a pharmaceutical preparation for the preventive anti-infective therapy following an acute stroke, wherein the therapy is started 12 to 72 hours following the stroke.

2. Use of an anti-infective agent according to claim 1, **characterized in that** the anti-infective agent contains one or more antibiotics in pharmaceutical preparation.

3. Use of an anti-infective agent according to claim 2, wherein the antibiotic or antibiotics can be selected from the classes of beta-lactam antibiotics, tetracyclines, aminoglykosides, lincosamines, glykopeptides, macrolides, carbapenemes, oxazolidinones, streptogramines and/or fluorochinolones.

4. Use of an anti-infective agent according to claim 2 or 3, wherein the antibiotic or antibiotics can be selected from the classes of beta-lactam antibiotics, carbapenemes, and/or the fluorochinolones.

5. Use of an anti-infective agent according to any of claims 1 to 4, **characterized in that** moxifloxacin *(1-Cyclopropyl-6-fluor-1,4-dihydro-8-methoxy-7-[(4aS, 7aS)-octahydro-6H pyrrolo[3,4-b]-pyridin-6-yl]-4-oxochinolin-3-carbon-säure)* is contained.

6. Use of an anti-infective agent according to claim 3 or 4 for producing a pharmaceutical preparation, wherein mezlocilline and sulbactam are used in combination.

7. Use of an anti-infective agent according to any of claims 1 to 6 for producing of agents and/or pharmaceutical preparations for a preventive anti-infective therapy following an acute stroke in mammals, particularly in domestic animals and livestock, in particular in humans.

8. Use of an anti-infective agent according to any of claims 1 to 7 for a protection from infections, such as pneumonia, urinary infections, and/or sepsis following an acute stroke.

9. Use of an immunomodulator according to claim 1, **characterized in that** the immunomodulator is selected from the group comprising cytokines and/or inhibitors of the SNS or endotoxin-binders or parapox-ovis-viral particles.

10. Use according to claim 8, wherein propranolol and/or IFN-γ is/are used.

11. Use according to any of claims 7 to 9 for producing agents and/or pharmaceutical preparations for a preventive anti-infective therapy following an acute stroke in mammals, particularly in domestic animals and livestock, in particular in humans.

12. Use of one or more anti-infective agents and one or more immunomodulators for producing a pharmaceutical preparation for a preventive anti-infective therapy following an acute stroke according to any of the above claims, wherein the anti-infective agent is selected from the classes comprising beta-lactam antibiotics, tetracyclines, aminoglykosides, lincosamines, glykopeptides, macrolides, carbapenemes, oxazolidinones, streptogramines as well as fluorochinolones, and the immunomodulator is selected from the group comprising cytokines (interleukins, interferons), inhibitors of the sympathic nervous system (beta-blockers, alpha-sympatho mimetics), endotoxin-binders, and inactivated parapoxwis-viral particles.

13. Use according to claim 12, wherein the anti-infective agent is moxifloxacin, and the immunomodulator is IFN-γ.

## Revendications

1. Utilisation d'un ou plusieurs agents anti-infectieux et/ou d'un ou plusieurs immunomodulateurs pour produire un agent et/ou une préparation pharmaceutique pour la thérapie anti-infectieuse préventive à la suite d'un accident cérébrovasculaire grave, où la thérapie est débutée 12 à 72 heures après l'accident cérébrovasculaire.

2. Utilisation d'un agent anti-infectieux selon la revendication 1, **caractérisé en ce que** l'agent anti-infectieux contient un ou plusieurs antibiotiques dans une préparation pharmaceutique.

3. Utilisation d'un agent anti-infectieux selon la revendication 2, où l'antibiotique ou les antibiotiques peuvent être sélectionnés à partir des classes d'antibiotiques de bêta-lactamines, de tétracyclines, d'aminoglucosides, de lincosamines, de glucopeptides, de macrolides, de carbapénèmes, d'oxazolidones, de streptogramines et/ou de fluorochinolones.

4. Utilisation d'un agent anti-infectieux selon la revendication 2 ou 3, où l'antibiotique ou les antibiotiques peuvent être sélectionnés à partir des classes d'antibiotiques de bêta-lactamines, de carbapénèmes et/ou de fluorochinolones.

5. Utilisation d'un agent anti-infectieux selon l'une quelconque des revendications 1 à 4, **caractérisé en ce que** de la moxifloxacine *(1-cyclopropyl-6-fluor-1, 4-dihydro-8-méthoxy-7-[(4aS, 7aS)-octahydro-6H-pyrrolo[3,4-b]-pyridin-6-yl]-4-oxochinolin-3-carbon-acide)* est contenue.

6. Utilisation d'un agent anti-infectieux selon la revendication 3 ou 4 destiné à produire une préparation pharmaceutique, où de la mezlocilline et du sulbactam sont utilisés en combinaison.

7. Utilisation d'un agent anti-infectieux selon l'une quelconque des revendications 1 à 6 destiné à produire des agents et/ou des préparations pharmaceutiques pour une thérapie anti-infectieuse préventive suivant un accident cérébrovasculaire grave chez des mammifères, particulièrement chez les animaux domestiques et le bétail, en particulier chez les humains.

8. Utilisation d'un agent anti-infectieux selon l'une quelconque des revendications 1 à 7, destiné à une protection contre des infections, telles qu'une pneumonie, des infections urinaires, et/ou une septicémie après un accident cérébrovasculaire.

9. Utilisation d'un immunomodulateur selon la revendication 1, **caractérisé en ce que** l'immunomodulateur est sélectionné à partir du groupe constitué de cytokines et/ou d'inhibiteurs du système nerveux sympathique (SNS) ou de liants d'endotoxine ou de particules de parapox-ovis-viral.

10. Utilisation selon la revendication 8, dans laquelle du propanol et/ou un IFN-γ est/sont utilisés.

11. Utilisation selon l'une quelconque des revendications 7 à 9 destinée à produire des agents et/ou des préparations pharmaceutiques pour une thérapie anti-infectieuse préventive suivant un accident cérébrovasculaire grave chez des mammifères, particulièrement chez les animaux domestiques et le bétail, en particulier chez les humains.

12. Utilisation d'un ou plusieurs agents anti-infectieux et d'un ou plusieurs immunomodulateurs pour produire une préparation pharmaceutique destinée à une thérapie anti-infectieuse préventive à la suite d'un accident cérébrovasculaire grave conformément à l'une quelconque des revendications ci-dessus, où l'agent anti-infectieux est sélectionné à partir des classes comprenant des antibiotiques de bêta-lactamines, des tétracyclines, des aminoglucosides, des lincosamines, des glucopeptides, des macrolides, des carbapénèmes, des oxazolidones, des streptogramines de même que des fluorochinolones, et l'immunomodulateur est sélectionné à partir du groupe constitué de cytokines (interleukines, interférons), d'inhibiteurs du système nerveux sympathique (des bêta-bloquants, des alpha-sympatho mimétiques), des liants d'endotoxine, et des particules inactivées de parapox-ovis-viral.

13. Utilisation selon la revendication 12, dans laquelle l'agent anti-infectieux est de la moxifloxacine et l'immunomodulateur est un IFN-γ.
